# EUROPEAN PATENT SPECIFICATION

(11) **EP 2 535 036 B1**
(45) Date of publication and mention of the grant of the patent: **29.11.2017**
(21) Application number: 11742229.5
(22) Date of filing: 08.02.2011
(51) Int. Cl.: A61K 8/02, A61K 8/04, A61Q 5/08, A61Q 5/10, A45D 34/00

(54) **DYEING OR BLEACHING KIT**
FÄRBUNGS- ODER BLEICHUNGSKIT
NÉCESSAIRE DE COLORATION OU DE DÉCOLORATION

(30) Priority: 21.04.2010 JP 2010097950; 10.02.2010 JP 2010028236
(43) Date of publication of application: 19.12.2012
(73) Proprietor: Kao Corporation, Chuo-ku Tokyo 103-8210 (JP)
(72) Inventor: MIYABE, Hajime, Tokyo 131-8501 (JP); YAMAGUCHI, Masakazu, Tokyo 131-8501 (JP); KANDA, Takashi, Tokyo 131-8501 (JP); YAMADA, Takashi, Tokyo 131-8501 (JP); KODAMA, Daisuke, Tokyo 131-8501 (JP)
(74) Representative: Hoffmann Eitle
(86) International application number: PCT/JP2011/052655
(87) International publication number: WO 2011/099485

(56) References cited:
- EP-A1- 1 470 812
- EP-A1- 2 062 562
- EP-A1- 2 500 010
- DE-A1- 4 018 259
- JP-A- 9 322 864
- JP-A- 2002 152 356
- JP-A- 2003 518 028
- JP-A- 2005 194 205
- JP-A- 2008 208 033
- JP-A- 2009 108 052
- JP-A- 2009 120 605
- JP-A- 2009 154 884
- JP-A- 2010 006 803
- DATABASE GNPD [Online] MINTEL; February 2010 (2010-02), "Shaking whipped hair colour", XP002740766, Database accession no. 1294574

## Description

### Field of the Invention

The present invention relates to a kit for dyeing or bleaching.

### Background of the Invention

Conventionally, a two-part hair bleach agent or hair dye has long been widely available in the form of liquid or cream. However, it is difficult for those who are not accustomed to using such a product to evenly apply it onto the hair. This is because the viscosity of a mixture to be applied onto the hair is adjusted relatively high so as to prevent dripping while the mixture is left on the hair, which makes it difficult to evenly spread the mixture and to allow the mixture to reach deep into the hair root. Furthermore, skill such as blocking and two-mirror technique is required for application of the mixture to the hair root and to the back of the head, which also requires a longer time.

In regard to this, simplification of hair dyeing operation by discharging the agent in the form of foam is proposed. For example, a product having a discharging container composed of two connected aerosol cans, which separately discharges a first part and a second part of a two-part hair dye in the form of foam (refer to Patent Document 1) and a product having a non-aerosol foamer container discharging a mixture of a two-part hair bleach agent or two-part hair dye in the form of foam (refer to Patent Document 2) are known.

Although a product having a discharging container composed of two connected aerosol cans, as described in Patent Document 1, can easily form foam, the first and second parts tend to form an uneven mixture because a first part and a second part are each discharged from independent aerosol cans in the form of foam, which results in uneven bleaching or dyeing. Moreover, the foam discharged from an aerosol can is formed by vaporization of a propellant, therefore, in the case of conventionally known aerosol-type formulations, once the foam is crushed, it is difficult to re-foam the foam. Thus, attempt to simplify the hair dyeing operation by intentionally forming foam from agent is meaningless. Furthermore, the use of an aerosol can is disadvantageous because of complexity of the structure of the container.

Also, by discharging a liquid mixture of a first part and a second part from a non-aerosol foamer container in the form of foam, a product having a non-aerosol foamer container discharging a mixture of a two-part hair bleach agent or two-part hair dye in the form of foam such as ones described in Patent Document 2 allows even those who are not accustomed to using such a product to apply the liquid mixture simply and evenly onto the hair, leading to an evenly-colored finish. Since such a product can be easily applied, skill such as blocking and two-mirror technique is not required, and time required for hair dyeing has been considerably reduced compared to conventional methods. Since the foam once applied can be re-foamed, the advantage of hair dyeing by using foam can be fully obtained. Since this product provides far superior performance compared to the conventional products, it is gaining popularity among a wide range of customers, regardless of sex and age groups.

However, in the case of using a non-aerosol foamer container as described in Patent Document 2, although the structure of the non-aerosol foamer container is simpler than that of an aerosol type container, it is still to some degree complex. Also, in order to discharge a liquid mixture from a non-aerosol foamer container in the form of foam, the viscosity of the liquid mixture is adjusted to 1 to 300 mPa·s, which is extremely low compared to the traditional liquid or cream products. Accordingly, further research and development for providing a formulation which retains discharging property from a non-aerosol foamer container while retaining basic performance for hair bleaching or dyeing, as well as paying attention to storage stability at a low or high temperature before use, would be necessary. Accordingly, because a hair bleaching or dyeing agent for use in discharging a liquid mixture from a non-aerosol foamer container in the form of foam is required to possess completely different physical properties from a traditional liquid or cream type hair bleaching or dyeing agents, the formulations used in these conventional products could not be directly applied.

### Prior Art Documents

### Patent Documents

[Patent Document 1] JP-A-H10-287534
[Patent Document 2] JP-A-2004-339216 (corresponding to EP 1470812)

DE 4018259 concerns aqueous hydrogen peroxide preparations for dyeing and bleaching the hair, comprising a specific content of anionic surfactants and a thickening polymer, which comprises carboxyl groups and is soluble in aqueous alkali.

EP 2062562 seeks to improve the foam quality and discharge properties of a two-part hair cosmetic for hair dyeing or bleaching by using a squeeze container to discharge a mixed solution of first and second agent of the kit in a foam.

JP 2000-0403993 describes an anti-slip seal for a portable telephone which reduces a possibility that a hand is slipped mistakenly so as to drop the telephone when the portable telephone is used.

EP 2500010 is a post-published patent application pursuant to Article 54(3) EPC and describes a cosmetic preparation for hair constituted as a hair dye or an agent for bleaching or removing dye from hair comprising a plurality of agents. The preparation is applied to the hair in the form of a foam obtained by mixing a powdered agent and a liquid agent and creating a foam through shaking.

### Summary of the Invention

The present invention provides a kit for hair dyeing or bleaching which includes: a first part containing an alkali agent; a second part containing an oxidizing agent; a thickening composition containing a thickening agent; and an openable and closable airtight container into which the first and second parts and the thickening composition are charged so as to form foam of a mixture thereof by shaking,
wherein any one or more of the first part, the second part, and the thickening composition comprise a surfactant, and wherein the inner circumference of the opening of the main body of the container is 25 to 35 cm.

The present invention also provides a kit for hair dyeing or bleaching which includes: a first part containing an alkali agent; a second part containing an oxidizing agent; and an openable and closable airtight container into which the first and second parts are charged so as to form foam of a mixture thereof by shaking, and wherein the airtight container is equipped with anti-slip means for prevention of slipping in both longitudinal and transverse directions on the surface thereof,
wherein any one or more of the first part and the second part comprise a surfactant, and wherein the inner circumference of the opening of the main body of the container is 25 to 35 cm.

The present invention also provides a method for dyeing or bleaching hair, which includes the following steps (a) to (d):
(a) charging all of a first part containing an alkali agent, a second part containing an oxidizing agent, and a thickening composition containing a thickening agent into a main body of an openable and closable airtight container,
(b) hermetically sealing the airtight container,
(c) forming foam by shaking the airtight container, and
(d) taking out the formed foam from the airtight container and applying the foam to hair,
wherein any one or more of the first part, the second part, and the thickening composition comprise a surfactant, and wherein the inner circumference of the opening of the main body of the container is 25 to 35 cm.

The present invention also provides a method for dyeing or bleaching hair, which includes the following steps (a) to (d):
(a) charging both of a first part containing an alkali agent and a second part containing an oxidizing agent into a main body of an openable and closable airtight container having anti-slip means for prevention of slipping in both longitudinal and transverse directions on the surface thereof,
(b) hermetically sealing the airtight container,
(c) forming foam of a liquid mixture by shaking the airtight container, and
(d) taking out the formed foam from the airtight container and applying the foam to hair,
wherein any one or more of the first part and the second part comprise a surfactant, and wherein the inner circumference of the opening of the main body of the container is 25 to 35 cm.

### Brief Description of the Drawings

[Figure 1] Figure 1 is a diagram showing an embodiment in which the airtight container has a dome-shaped hermetically sealed lid.
[Figure 2] Figure 2 is a diagram showing an embodiment in which the airtight container constitutes duplex structure, and (a) is a longitudinal sectional view of the airtight container constituting duplex structure and (b) is a perspective view of the airtight container constituting duplex structure.
[Figure 3] Figure 3 is a diagram showing a state in which the airtight container constituting duplex structure is in use.
[Figure 4] Figure 4 is a diagram showing an embodiment in which the airtight container used in the present invention has an accordion structure, and (a) shows a contracted state while (b) shows an expanded state.
[Figure 5] Figure 5 is a diagram showing an embodiment in which an airtight container having an accordion structure has a hermetically sealed lid with a discharge port.
[Figure 6] Figure 6 is a diagram showing specific examples of anti-slip means in an airtight container used in the present invention.

### Embodiment for Carrying out the Invention

The present invention relates to a kit for dyeing or bleaching which satisfies all of the following needs at the same time:
1) structure of the container is simple,
2) foam can be easily formed,
3) formulation can be prepared freely,
4) the advantage of the foamy hair dye of Patent Document 2, which enables even those who are not accustomed to using the product to apply it easily and evenly onto the hair and obtain an evenly-colored finish, is also obtained,
5) storage stability of the agent is excellent,
6) basic performance as a hair dye, for example, dyeing can be preferably provided.

The present inventors have found that all of the above needs are successfully satisfied by a kit for dyeing or bleaching using a specific container and a method for dyeing or bleaching hair including specific steps.

### [Embodiments of the kit for dyeing or bleaching]

The embodiments of the kit for dyeing or bleaching of the present invention include a two-part type kit and a three-part type kit.
- Examples of the two-part type kit include:
   - an embodiment composed of a first part containing an alkali agent and a thickening agent and a second part containing an oxidizing agent, and
   - an embodiment composed of a first part containing an alkali agent and a second part containing an oxidizing agent and a thickening agent.
- Examples of the three-part type kit include:
   - an embodiment composed of a first part containing an alkali agent, a second part containing an oxidizing agent, and a thickening composition containing a thickening agent,
   - an embodiment composed of a first part containing an alkali agent and a thickening agent, a second part containing an oxidizing agent, and a thickening composition containing a thickening agent, and
   - an embodiment composed of a first part containing an alkali agent, a second part containing an oxidizing agent and a thickening agent, and a thickening composition containing a thickening agent.

The aforementioned first part and second part may be in any of the conventionally known forms such as a liquid form, a gel form, a jelly form, a powdery form, a granular form, a cream form, and a molded product (such as a tablet).

The molded product may be in any geometric structure; however, from the viewpoint of easy operation when introducing it into an airtight container and dissolving it by shaking, as well as its role as a stirrer, the mass of one molded product is preferably 1 to 20 g, more preferably 1.5 to 10 g, and even more preferably 2 to 8 g.

The kit for dyeing or bleaching may contain additional agents such as an oxidation aid such as persulfate, an auxiliary agent such as animal and plant extracts and fragrance, a component (medium) for diluting the alkali agent, the oxidizing agent, and the thickening agent, a pre-treatment agent for use before hair dyeing, and a post-treatment agent for use after hair dyeing. In the present specification, the kit for dyeing or bleaching additionally containing these auxiliary agents and treatment agents are also regarded as a two-part type kit, a three-part type kit, or a complex molded product type kit. The term "liquid mixture" in the present specification hereinafter means a liquid mixture of all the components to be charged into the airtight container, unless otherwise specifically defined.

As embodiments of the kit for dyeing or bleaching of the present invention, any of the following embodiments is preferred:
- A two-part type kit composed of a first part containing an alkali agent and a thickening agent, and a second part containing an oxidizing agent,
- A three-part type kit composed of a first part containing an alkali agent, a second part containing an oxidizing agent, and a thickening composition containing a thickening agent, and

### [Hair]

In the present invention, the hair may be hair grown on the head (head hair) or hair grown elsewhere, preferably head hair. Also, although any kind of hair, such as hair of a doll or animal hair, may be used, human head hair is preferred.

### [Thickening agent]

In the present invention, the thickening agent refers to a component which increases the viscosity of a liquid mixture of the first part and the second part, a liquid mixture of the first part, the second part, and the thickening composition, or a liquid mixture prepared from the molded product and the medium. In the case of a two-part type kit or a three-part type kit, the thickening agent may be contained in either the first part or the second part. Alternatively, the thickening agent may also be contained in the thickening composition separately from the first part and the second part.

One or more members selected from the group consisting of a synthetic polymer, a semi-synthetic polymer, and a natural polymer may be used as the thickening agent.

Examples of the synthetic polymer include a carbomer (for example, Sumitomo Seika Chemicals Co., Ltd., Aqupec HV-501), an acrylate copolymer (for example, Rohm and Haas Company, Aculyn 33 polymer, BASF, Luviflex Soft), a (PEG-150/stearyl alcohol/SMDI) copolymer (for example, Rohm and Haas Company, Aculyn 46 and Aculyn 46B), a dimethyldiallylammonium chloride/acrylamide copolymer (for example, Nalco Company, Merquat 100 and Merquat 550), and a vinylpyrrolidone/dimethylamino ethyl methacrylate copolymer (for example, International Specialty Products (ISP) Inc., copolymer 845, copolymer 937, and copolymer 958).

Examples of the semi-synthetic polymer include hydroxyethyl cellulose o-[2-hydroxy-3-(lauryldimethylammonio)propyl] chloride (for example, Dow Chemical Japan Ltd., UCARE Polymer JR-125, JR-30M, and JR-400, and Lion Corporation, Leogard G), o-[2-hydroxy-3-(trimethylammonio)propyl] guar gum chloride (for example, Dainippon Sumitomo Pharma Co., Ltd., Rhaball Gum CG-M, Rhaball Gum CG-6L, Rhaball Gum CG-M7, Rhaball Gum CG-M8M, Rhodia, Jaguar C-13S, Jaguar C-14S, Jaguar C-17, Jaguar C-210, Jaguar C-162, and HI-CARE1000), cationic dextran, methyl cellulose (for example, Shin-Etsu Chemical Co., Ltd., Metolose SM), hydroxyethyl cellulose (for example, Dow Chemical Japan Ltd., Cellosize QP4400H and QP52000H, Daicel Corporation, SE-600 and SE-850), hydroxypropyl cellulose (for example, Nippon Soda Co., Ltd., Nisso HPC-H, HPC-M), hydroxypropylmethyl cellulose (for example, Shin-Etsu Chemical Co., Ltd., Metolose 60SH series, Metolose 65SH series, and Metolose 90SH series), hydroxypropyl xanthan gum (for example, Dainippon Sumitomo Pharma Co., Ltd., Rhaball GumEX), and pullulan fatty acid esters.

Examples of the natural polymer include pullulan (for example, Hayashibara Shoji Inc., Pullulan PF-20 and pullulan PI-20), xanthan gum (for example, Dainippon Sumitomo Pharma Co., Ltd., Echo gum), agar (for example, Ina Food Industry Co., Ltd., Ina agar CS), gellan gum (for example, Dainippon Sumitomo Pharma Co., Ltd., Kelco gel), collagen (for example, Koken Co., Ltd., Mariblen Mask SH), alginic acid (for example, Dainippon Sumitomo Pharma Co., Ltd., Alginic acid HFD), gum arabic (for example, Ina Food Industry Co., Ltd., Ina gel gum arabic CS), guar gum (for example, Ina Food Industry Co., Ltd., Ina gel guar gum CS), and locust bean gum (Ina Food Industry Co., Ltd., Ina gel locust bean gum CS).

As described above, any of the first part, the second part, and the thickening composition may contain a thickening agent, and the thickening agent-containing composition may be in any form such as a liquid form, a gel form, a jelly form, a powdery form, a granular form, a cream form, and a molded product. However, from the viewpoint of avoiding uneven dyeing due to the production of "lump" during mixing, a liquid form, a gel form, a jelly form, and a cream form are preferred, among them, a liquid form, a gel form, and a jelly form are more preferred. Furthermore, a gel form and a jelly form are preferred, from the viewpoint that the foam may be easily formed by preventing increase of the viscosity of a liquid mixture at initiation of shaking, while from the viewpoint that the formed foam may be stabilized by increasing the viscosity of a liquid mixture upon completion of shaking due to gradual disintegration and dissolution of the "mass" while shaking in another liquid composition at the initial stage of mixing.

Also, from the viewpoint of preventing the production of "lump" during mixing and easily forming foam by preventing increase of viscosity of a liquid mixture at initiation of shaking, while from the viewpoint of stabilizing the formed foam by increasing the viscosity of a liquid mixture upon completion of shaking, the composition containing thickening agent is preferably a composition containing water or a water soluble organic solvent. Examples of the water soluble organic solvent include lower alkanols such as ethanol and 2-propanol; and lower polyols such as propylene glycol, 1,3-butanediol, diethylene glycol, and glycerin. Among them, a composition containing water or ethanol is preferred. The content of water or a water soluble organic solvent is preferably 10 to 99.5% by mass, more preferably 20 to 95% by mass, and even more preferably 30 to 90% by mass of the thickening agent-containing composition.

That is, in the case that the thickening agent-containing composition in a gel form or a jelly form is mixed with the other agents,
1) at the stage when each agent is simply charged into the main body of an airtight container, the mixture is in such a mixed state that the part derived from the composition containing a thickening agent component (in a gel form or a jelly form) is distinguishable from the remaining part (in a liquid form), and
2) when an external force is applied to the mixture by shaking the airtight container, the part derived from the composition containing a thickening agent component (in a gel form or a jelly form) gradually becomes smaller through disintegration and dissolution, and a preferable mixed state is obtained at last.

Herein, the term "gel form" commonly refers to a state in which colloid particles, which are the components of the composition, interact with each other by forming bonds to form three-dimensional network structure, and the matrix is filled with liquid. Meanwhile, the term "jelly form" refers to simply a semisolid state of the "gel form", which is resistant against weak external force such as touching by hand, however disintegrates by stronger external force. However, the concepts of the "gel form" and "jelly form" should not be strictly distinguished from each other.

When the thickening agent-containing composition is in a powdery form or a granular form, it is an aggregate of minute solids. When the thickening agent-containing composition is in a liquid form, gel form, or cream form, the viscosity thereof is preferably more than 200 mPa·s and 100,000 mPa·s or less, more preferably 500 to 80,000 mPa·s, and even more preferably 1,000 to 50,000 mPa·s. When the thickening agent-containing composition is a jelly composition, the strength of the jelly is preferably 5 to 1000 g, more preferably 10 to 500 g, and even more preferably 50 to 350 g.

Here, the viscosity refers to a viscosity as measured by using TVB-10M model viscometer (Toki Sangyo Co., Ltd.), at 25°C, rotating for one minute. The measurement is performed employing following combinations of rotor and rotation speed, according to the following respective cases.
- 500 mPa·s or less: rotor: M2, rotation: 60 rpm
- More than 500 mPa·s and 4,000 mPa·s or less: rotor: M3, rotation: 30 rpm
- More than 4,000 mPa·s and 10,000 mPa·s or less: rotor: M3, rotation: 12 rpm
- More than 10,000 mPa·s and 100,000 mPa·s or less: rotor: M4, rotation: 6 rpm.

Also, the strength of jelly is measured in accordance with JIS K6503: 2001 standard. Conditions for measurement: using a texture analyzer (TA-1000 texture analyzer, Kett Electric Laboratory); 120 g of a sample is placed flatly in a jelly cup with an inner diameter of 60 mm and a height of 60 mm, without trapping air; sample temperature: 20°C, a probe penetration distance: 4 mm, and a probe penetration speed: 1 mm/s.

Also, the viscosity (25°C) of the compositions other than the thickening agent-containing composition is preferably 1 to 200 mPa·s, more preferably 2 to 120 mPa·s, and even more preferably 5 to 80 mPa·s, from the viewpoint of manageability and convenience of formation of foam.

Further, the viscosity (25°C) of the liquid mixture may be higher than that of the remaining compositions other than the thickening agent-containing composition. From the viewpoint of easily forming foam at a low viscosity in the initial stage of shaking, while once the foam is formed, from the viewpoint of stabilizing the formed foam by increasing the viscosity, the viscosity (25°C) of the liquid mixture is preferably 1.2 times or more, preferably 2 times or more, and more preferably 10 times or more higher than the viscosity of the remaining components other than the thickening agent-containing composition. Further, from the viewpoint of allowing the formed foam to easily reach into the root of the hair upon application and to easily spread from the root to the tip of the hair, the viscosity (25°C) of the liquid mixture is preferably 50 to 20,000 mPa·s, preferably 500 to 10,000 mPa·s, and more preferably 1,000 to 5,000 mPa·s. The viscosity of the liquid mixture as referred to herein means not the viscosity of the foam of the liquid mixture formed by shaking the airtight container but the viscosity of a solution obtained by mixing under the following conditions: a solution is obtained by weighing out a total of 50 g of all the agents at a certain ratio and charging them in a 100 mL beaker (diameter of 55 mm, height of 70 mm), followed by stirring with a magnetic stirrer (the stirrer has an octagonal shape: total length of 40 mm, diameter of 10 mm) at 600 rpm for one minute, and leaving the resulting mixture to stand. The viscosity of 10 minutes after introducing the agents is defined as the viscosity of the liquid mixture. As long as the viscosity is within the above range after 10 minutes, even when the viscosity is reduced over time scale of several tens of minutes, the length of which is usual length for leaving a hair dye to stand, there is less possibility of dripping, while even when the viscosity is increased, as long as the foam is maintained as foam, it is easily spread from the root to the tip of the hair.

As described above, various kinds of components may be mixed in a hair dye or bleaching agent. As long as an appropriate viscosity can be obtained 10 minutes after the initiation of mixing, it does not matter if a hair dye contains a component inhibiting the thickening action of a thickening agent. Thus, a thickening agent can be selected from a wide range of the aforementioned viscosity increasing agents.

### [Alkali agent]

An alkali agent is contained in the first part of the kit for dyeing or bleaching of the present invention. Examples of the alkali agent include ammonia and a salt thereof (for example, ammonium carbonate and ammonium bicarbonate); ethanolamine and a salt thereof (for example, monoethanolamine and diethanolamine); alkanolamine other than ethanolamine and a salt thereof such as isopropanolamine, 2-amino-2-methyl propanol, and 2-aminobuatnol; alkanediamine and a salt thereof such as 1,3-propanediamine; carbonate such as guanidine carbonate, sodium carbonate, potassium carbonate, guanidine bicarbonate, sodium bicarbonate, and potassium bicarbonate. Among them, ammonia, monoethanolamine, and ammonium bicarbonate are preferred. A combination of two or more of these alkali agents may be used, and from the viewpoint of sufficient bleaching and hair dyeing effects and reduction of hair damage and pungent odor during hair dyeing or bleaching operation, it is preferable to use ammonia or a salt thereof in combination with ethanolamine or a salt thereof.

From the viewpoint of sufficient bleaching and hair dyeing effects as well as reduction of hair damage and scalp irritation, the content of these alkali agents is preferably 0.01 to 15% by mass, more preferably 0.1 to 10% by mass, and even more preferably 0.2 to 5% by mass of the liquid mixture.

Further, from the viewpoint of obtaining sufficient hair dyeing and bleaching properties, and of enabling selection of a thickening agent from a wide range of the aforementioned thickening agents, the pH of the liquid mixture is preferably 7 to 12, more preferably 8 to 11, and even more preferably 8.5 to 10.5.

### [Oxidizing agent]

An oxidizing agent is contained in the second part of the kit for dyeing or bleaching of the present invention. Examples of the oxidizing agent include hydrogen peroxide and a hydrogen peroxide-producing agent such as urea peroxide, melamine peroxide, sodium perborate, potassium perborate, sodium percarbonate, and potassium percarbonate. When the second part is a liquid, hydrogen peroxide is preferred, and when the second part is a solid or the oxidizing agent is contained in the molded product, urea peroxide is preferred.

A combination of two or more of the oxidizing agents may be used, and from the viewpoint of sufficient bleaching and hair dyeing effects as well as reduction of hair damage and scalp irritation, the content of the oxidizing agent is preferably 0.1 to 10% by mass, more preferably 1 to 5% by mass of the liquid mixture, in terms of the hydrogen peroxide equivalent.

### [Surfactant]

A surfactant is contained in any one or more of the first part, the second part, and the thickening composition of the kit for dyeing or bleaching of the present invention. From the viewpoint of good lathering and easy re-foaming after application of foam to the hair, the total concentration of all the surfactants in the liquid mixture is preferably 0.01 to 20% by mass, more preferably 0.1 to 15% by mass, and even more preferably 1 to 10% by mass.

Examples of the anionic surfactant include a sulfuric acid ester surfactant such as alkyl sulfate and polyoxyalkylene alkyl ether sulfate; a carboxylic acid surfactant such as a fatty acid salt, an N-acyl amino acid salt (such as an N-acyl sarcosine salt, an N-acyl glutamic acid salt, and an N-acyl glycine salt), a salt of alkyl succinate or alkenyl succinate, polyoxyalkylene alkyl ether carboxylate, and fatty acid amide ether acetate; a phosphoric acid ester surfactant such as alkyl phosphate and alkyl ether phosphate; and a sulfonic acid surfactant such as polyoxyalkylene alkyl ether sulfosuccinate, an isethionic acid fatty acid ester salt, an acyl taurine salt, alkyl benzene sulfonic acid, α-olefin sulfonic acid, and alkane sulfonic acid. Among them, alkyl sulfate and polyoxyalkylene alkyl sulfate are preferred, more preferably, the alkyl group thereof having 10 to 24 carbon atoms, even more preferably, having 12 to 18 carbon atoms, even more preferably, the alkyl group being a linear alkyl chain. Also, polyoxyalkylene alkyl sulfate, especially polyoxyethylene alkyl sulfate is preferred, more preferably having an average molar number of oxyethylene groups added of 1 to 10, even more preferably having an average molar number of oxyethylene groups added of 2 to 5. Further, N-acyl amino acid salt and ether carboxylic acid salt are also preferred, and N-acyl glutamic acid salt having an acyl group having 10 to 18 carbon atoms and polyoxyethylene alkyl carboxylate having an alkyl group having 10 to 18 carbon atoms and an average molar number of oxyethylene groups added of 3 to 15 are preferred. A combination of two or more of anionic surfactants can be used, and the content thereof is preferably 0.01 to 15% by mass, more preferably 0.1 to 10% by mass, and even more preferably 1 to 5% by mass of the liquid mixture.

Further, an amphoteric surfactant, a nonionic surfactant, a cationic surfactant, and a semi polar surfactant can also be used.

Examples of the amphoteric surfactant include carbobetaine-, amidobetaine-, sulfobetaine-, hydroxyl sulfobetaine-, amidosulfobetaine-, phosphobetaine-, and imidazolinium-based surfactants, all of which have an alkyl group, an alkenyl group, or an acyl group having 8 to 24 carbon atoms, among which carbobetaine- and sulfobetaine-based surfactants are preferred. Examples of a preferred amphoteric surfactant include lauramidopropyl betaine, coconut oil fatty acid amidopropyl betaine, lauryldimethylaminoacetic acid betaine, and laurylhydroxysulfobetaine. A combination of two or more of amphoteric surfactants can be used, and the content thereof is preferably 0.01 to 15% by mass, more preferably 0.1 to 10% by mass, and even more preferably 1 to 5% by mass of the liquid mixture.

Examples of the nonionic surfactant include an alkyl polyglucoside, a polyoxyalkylene alkyl ether, and an alkyl glyceryl ether. In an alkyl polyglucoside, the number of carbon atoms in the alkyl group is preferably 8 to 18, more preferably 8 to 14, and even more preferably 9 to 11, and the alkyl group is preferably linear. The average degree of condensation of the glucoside is preferably 1 to 5, more preferably 1 to 2. In a polyoxyalkylene alkyl ether, the number of carbon atoms in the alkyl group is preferably 10 to 22, more preferably 12 to 18, and the alkyl group is preferably linear. Also, polyoxyethylene alkyl ether is more preferred, and especially a polyoxyethylene alkyl ether having an average number of moles added of oxyethylene groups of 1 to 40 is preferred, and a polyoxyethylene alkyl ether having an average number of moles added of oxyethylene groups of 4 to 30 is more preferred. In an alkyl glyceryl ether, the number of carbon atoms in the alkyl group is preferably 8 to 18, more preferably 8 to 12, and the alkyl group is preferably branched. A combination of two or more of nonionic surfactants can be used, and the content thereof is preferably 0.01 to 15% by mass, more preferably 0.1 to 10% by mass, and even more preferably 1 to 5% by mass of the liquid mixture.

Cationic surfactant represented by, for example, the following general formula (1) can be used: 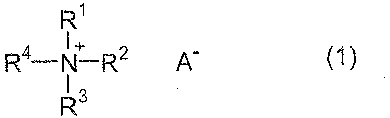 wherein, R¹, R², R³ and R⁴ are each independently an optionally substituted hydrocarbon group, at least one of R¹ and R² have 8 to 36 carbon atoms and the rest have 1 to 7 carbon atoms, or R³ and R⁴ together form with an adjacent nitrogen atom a 5- to 7-membered ring optionally substituted with an alkyl group having 1 to 4 carbon atoms and optionally having, besides the nitrogen atom, a nitrogen atom, an oxygen atom, and a sulfur atom as a hetero atom. A⁻ represents an anion.

Here, examples of the hydrocarbon group include a linear or branched-chain alkyl group, a linear or branched-chain alkenyl group, an aryl group, and an aralkyl group, and examples of the substituent include a hydroxy group, an alkoxy group, an aryloxy group, an epoxy group, an amino group, a mono- or di-alkylamino group, a trialkylammonium group, a fatty acid amide group, and a fatty acid ester group. Also, examples of the ring formed by R³ and R⁴ with an adjacent nitrogen atom include a morpholine ring, an imidazoline ring, a piperazine ring, a piperidine ring, and a pyrrolidine ring.

Examples of the anion include a chloride ion, a bromide ion, an iodide ion, a methyl sulfate ion, an ethyl sulfate ion, an acetate ion, a phosphate ion, a sulfate ion, a lactate ion, and a saccharin ion.

Specific examples of the cationic surfactant include cetyltrimethylammonium chloride, stearyltrimethylammonium chloride, isostearyltrimethylammonium chloride, lauryltrimethylammonium chloride, behenyltrimethylammonium chloride, octadecyltrimethylammonium chloride, cocoyltrimethylammonium chloride, cetyltrimethylammonium bromide, stearyltrimethylammonium bromide, lauryltrimethylammonium bromide, isostearyllauryldimethylammonium chloride, dicetyldimethylammonium chloride, distearyldimethylammonium chloride, dicocoyldimethylammonium chloride, γ-gluconamidopropyldimethylhydroxyethylammonium chloride, di[polyoxyethylene(2)]oleylmethylammonium chloride, dodecyldimethylethylammonium chloride, octyldihydroxyethylmethylammonium chloride, tri(polyoxyethylene(5))stearylammonium chloride, polyoxypropylenemethyldiethylammonium chloride, lauryldimethyl(ethylbenzyl)ammonium chloride, behenamidopropyl-N,N-dimethyl-N-(2,3-dihydroxypropyl)ammonium chloride, tallowdimethylammoniopropyltrimethylammonium dichloride, and benzalkonium chloride.

Preferable cationic surfactant includes a monoalkyltrimethylammonium salt and a dialkyldimethylammonium salt, namely the compounds in which R¹ or both of R¹ and R² are a linear or branched-chain alkyl group having 8 to 30 carbon atoms, further, 10 to 24 carbon atoms, and furthermore, 12 to 18 carbon atoms, and the rest is a methyl group, among which a monoalkyltrimethylammonium salt is more preferred.

A combination of two or more of cationic surfactants can be used, and the content thereof is preferably 0.1 to 5% by mass, more preferably 0.2 to 3% by mass, and even more preferably 0.3 to 1% by mass of the liquid mixture.

Also, from the viewpoint of obtaining sufficient foaming by shaking, increasing the stability of the formed foam, and allowing the foam to reach easily and deeply into the root of the hair upon application, the mass ratio of an anionic surfactant to a cationic surfactant (anionic surfactant / cationic surfactant) is preferably 1 to 100, more preferably 2 to 50, and even more preferably 4 to 20.

Examples of the semi polar surfactant include alkylamine oxide.

### [Dye]

When the product of the present invention is a bleaching kit, it does not contain a dye, whereas when the product of the present invention is a dyeing kit, the first part contains an oxidation dye intermediate or a direct dye.

### (Oxidation dye intermediate)

Publicly known precursors and couplers normally used in a hair dye can be used as the oxidation dye intermediate. Examples of the precursor include para-phenylenediamine, toluene-2,5-diamine, 2-chloro-para-phenylenediamine, N-methoxyethyl-para-phenylenediamine, N,N-bis(2-hydroxyethyl)-para-phenylenediamine, 2-(2-hydroxyethyl)-para-phenylenediamine, 2,6-dimethyl-para-phenylenediamine, 4,4'-diaminodiphenylamine, 1,3-bis(N-(2-hydroxyethyl)-N-(4-aminophenyl)amino)-2-propanol, PEG-3,3,2'-para-phenylenediamine, para-aminophenol, para-methylaminophenol, 3-methyl-4-aminophenol, 2-aminomethyl-4-aminophenol, 2-(2-hydroxyethylaminomethyl)-4-aminophenol, ortho-aminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraaminopyrimidine, 2,5,6-triamino-4-hydroxypyrimidine, 4,5-diamino-1-(4'-chlorobenzyl)pyrazole, 4,5-diamino-1-hydroxyethylpyrazole, and salts of these substances.

Also, examples of the coupler include meta-phenylenediamine, 2,4-diaminophenoxyethanol, 2-amino-4-(2-hydroxyethylamino)anisole, 2,4-diamino-5-methylphenetole, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis(2,4-diaminophenoxy)propane, meta-aminophenol, 2-methyl-5-aminophenol, 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-meta-aminophenol, 2-methyl-4-methoxy-5-(2-hydroxyethylamino)phenol, 2-methyl-4-fluoro-5-aminophenol, resorcin, 2-methylresorcin, 4-chlororesorcin, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine, 2,6-diaminopyridine, and salts of these substances.

Combination of two or more of the precursor and coupler can be used, and the content of each of them is preferably 0.01 to 5% by mass, more preferably 0.1 to 4% by mass of the liquid mixture.

### (Direct dye)

Examples of the direct dye include an acid dye, a nitro dye, a disperse dye, and a basic dye. Examples of the acid dye include Blue No. 1, Violet No. 401, Black No. 401, Orange No. 205, Red No. 227, Red No. 106, Yellow No. 203, and Acid orange No. 3. Examples of the nitro dye include 2-nitro-p-phenylenediamine, 2-amino-6-chloro-4-nitrophenol, 3-nitro-p-hydroxyethylaminophenol, 4-nitro-o-phenylenediamine, 4-amino-3-nitrophenol, 4-hydroxypropylamino-3-nitrophenol, HC Blue No.2, HC Orange No.1, HC Red No.1, HC Yellow No.2, HC Yellow No.4, HC Yellow No.5, HC Red No.3, and N,N-bis(2-hydroxyethyl)-2-nitro-p-phenylenediamine. Examples of the disperse dye include Disperse Violet 1, Disperse Blue 1, and Disperse Black 9. Examples of the basic dye include Basic Blue 99, Basic Brown 16, Basic Brown 17, Basic Red 76, Basic Red 51, Basic Yellow 57, Basic Yellow 87, and Basic Orange 31.

Combination of two or more of direct dyes can be used, and the direct dye can be used in combination with an oxidation dye intermediate. The content thereof is preferably 0.001 to 5% by mass, more preferably 0.01 to 3% by mass of the liquid mixture.

### [Oil agent]

In the kit for dyeing or bleaching of the present invention, from the viewpoint of imparting a firm foam quality to the foam of the liquid mixture and evenly dyeing from the root to the tip of the hair, a formulation is preferably formulated wherein the liquid mixture contains 0.01 to 3% by mass, more preferably 0.03 to 2.5% by mass, and even more preferably 0.05 to 2% by mass of an oil agent.

Examples of the oil agent include hydrocarbons such as squalene, squalane, liquid paraffin, liquid isoparaffin, and cycloparaffin; glycerides such as castor oil, cacao oil, mink oil, avocado oil, and olive oil; waxes such as bees wax, whale wax, lanoline, and carnauba wax; esters such as isopropyl palmitate, isopropyl myristate, octyldodecyl myristate, hexyl laurate, cetyl lactate, propylene glycol monostearate, oleyl oleate, hexadecyl 2-ethylhexanoate, isononyl isononanoate, and tridecyl isononanoate; higher fatty acids such as capric acid, lauric acid, myristic acid, palmitic acid, stearic acid, behenic acid, oleic acid, coconut fatty acid, isostearic acid, and isopalmitic acid; higher alcohols such as myristyl alcohol, cetyl alcohol, stearyl alcohol, behenyl alcohol, 2-octyldodecanol, and cetostearyl alcohol; and further, isostearyl glyceryl ether and polyoxypropylene butyl ether. Among them, higher alcohols are preferred, myristyl alcohol, cetyl alcohol, and stearyl alcohol are more preferred.

### [Medium]

Water and, if needed, an organic solvent are used in the liquid mixture of the present invention as a medium. Examples of the organic solvent include lower alkanols such as ethanol and 2-propanol; aromatic alcohols such as benzylalcohol and benzyloxyethanol; lower polyols such as propylene glycol, dipropylene glycol, 1,3-butanediol, diethylene glycol, and glycerin; cellosolves such as ethyl cellosolve, butyl cellosolve, and benzyl cellosolve; and carbitols such as ethyl carbitol and butyl carbitol. Such a media, in the case of a two-part type or three-part type kit, is usually contained in one of the parts. Thus, by mixing each of the parts, a liquid mixture can be prepared wherein each component at a desired concentration is contained. Meanwhile, when a product does not contain a medium, such as in the case of a complex molded product type kit, a liquid mixture containing each component at a desired concentration can be prepared by mixing with an adequate amount of medium.

### [Optional component]

In addition to the above-mentioned components, other components routinely used as cosmetic ingredients can be added to the first part, the second part or the thickening composition, used in the present invention. Examples of such an optional component include animal and plant oil and fat, silicone, ether, protein derivatives, protein hydrolysate, amino acids, preservatives, chelating agents, stabilizers, antioxidants, plant extracts, crude drug extracts, vitamins, fragrance, and ultraviolet ray absorbers.

### [Airtight container]

The airtight container may be in any form as long as foam of the contents can be prepared by shaking the container. For example, the airtight container may be composed of a main body of container and a hermetical sealing lid, wherein the main body of container and the hermetical sealing lid may be separable or inseparable (for example, the main body and the lid may be connected each other via hinge etc. in order to open and close the lid without separation of the lid from the main body of the airtight container).

In view of productivity, the main body of a container has preferably cylindrical shape with one of the bottom surfaces being open, and an anti-tip leg may be mounted on the other bottom surface. Examples of the cylindrical shape include columnar shape, conical shape, and double conical shape.

Radius from the central axis to at least a part of the side of the cylindrical shaped main body of the container is preferably 3 to 6 cm, more preferably 3.5 to 5.5 cm, and even more preferably 4 to 5 cm, in order to be easy to hold and shake with one hand, and in view of the volume of a foamed hair dye. Also, considering that the foamed hair dye is preferably scooped out by a hand, from the viewpoint of easily putting the hand into the container and easily opening and closing of the lid, the inner circumference of the opening is 25 to 35 cm, more preferably 26 to 34 cm, and even more preferably 27 to 33 cm. The height of the main body of a container is preferably 10 to 22 cm, more preferably 11 to 20 cm, and even more preferably 12 to 18 cm.

When the hair dye is foamed to the utmost limit, the volume of the foamed hair dye becomes equal to the capacity of the airtight container. Therefore, the whole capacity of the airtight container, i.e., total volume of the main body and volume part of the hermetical sealing lid, is preferably 100 to 2000 mL, more preferably 300 to 1500 mL, and even more preferably 500 to 1000 mL. Also, a ratio of the capacity of the airtight container (i.e., the total volume of the main body and volume part of the hermetical sealing lid) to the volume of the liquid mixture (the capacity of the airtight container / the volume of the liquid mixture), is preferably 3 to 10, more preferably 4 to 9, and even more preferably 5 to 8, from the viewpoint of preparing foam which is easily spread from the root to the tip of the hair, is hard to be dripped, and is easily formed by shaking.

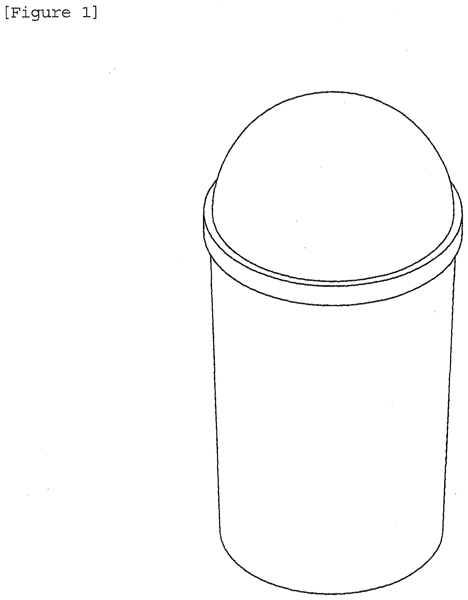

The hermetical sealing lid may be an outer lid which fits the container from outside or an inner lid which fits the inside of the container. Also, the shape of the hermetical sealing lid may be convex or concave shape toward the outside, or planar shape. From the viewpoint of easy foaming, it is preferably convex shape toward the outside, more preferably a dome shape. An embodiment of an airtight container in which the hermetical sealing lid is in a dome shape is shown in Figure 1.

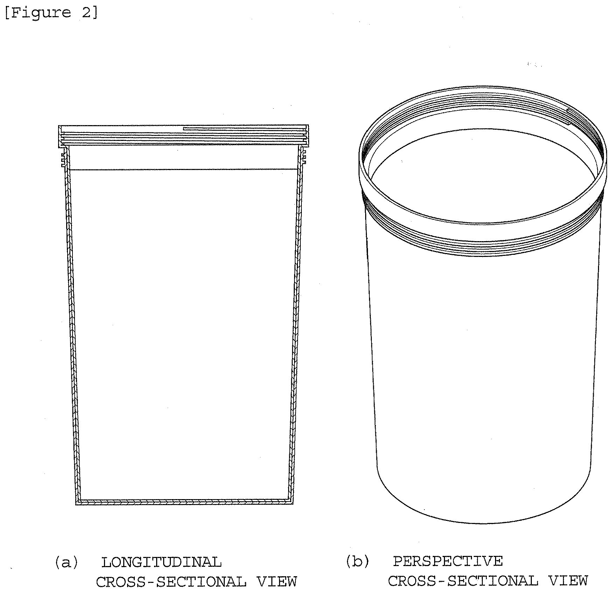

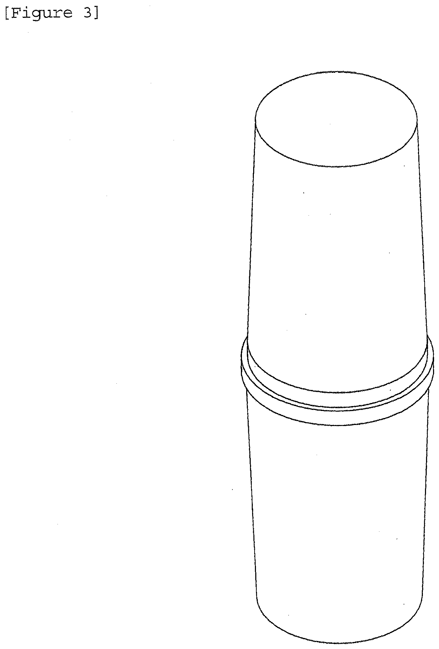

Also, as another embodiment, the airtight container may be in multiplex-structured shape where the main body is composed of two or more, preferably two members. The main body of a container is used as multiplex-structured shape in the process of distribution, storage, etc., whereas upon use, one or more of the members is used as a hermetically sealed lid. This embodiment is preferred because the volume of the overall container can be reduced in the process of distribution, storage, etc., while the capacity of the airtight container to be described later can be increased upon application. An embodiment of an airtight container having a duplex structure is shown in Figures 2 and 3. In Figure 2, (a) is a longitudinal sectional view of the airtight container having a duplex structure and (b) is a perspective view of the airtight container having an duplex structure. Figure 3 shows a state in which the airtight container having a duplex structure is in use.

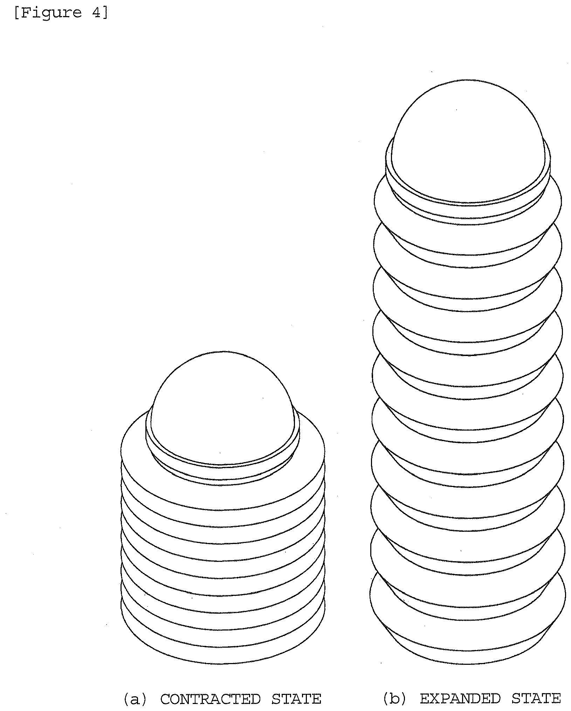

Also, the airtight container may have expandable and contractible means toward the depth direction. The airtight container is preferably expandable and contractible because in that way, the volume of the overall container can be reduced in the process of distribution, storage, etc., while the capacity of the airtight container can be increased upon application. Alternatively, the expandable and contractible airtight container is preferable because the foam inside the main body of a container can be easily taken out by contracting the expandable and contractible means when the remaining amount of foam in the airtight container is decreased upon application. Alternatively, the expandable and contractible airtight container is preferable because the foam inside the main body of a container can be discharged from the discharge port to be described later by contracting the expandable and contractible means upon application. Either one or both of the main body of a container and the hermetical sealing lid may have the expandable and contractible means toward the depth direction; however, from the viewpoint of easy handling, it is preferred that the main body of a container has the expandable and contractible means toward the depth direction. The expandable and contractible means toward the depth direction may be any means, an accordion structure is preferred because it can be expanded and contracted easily, while the airtightness of the container can be secured. An embodiment of an airtight container having an accordion structure as the expandable and contractible means is shown in Figure 4, in which (a) shows a contracted state and (b) shows an expanded state.

It should be noted that the capacity of an airtight container having the "expandable and contractible means" refers to a volume of the container when it is maximally expanded.

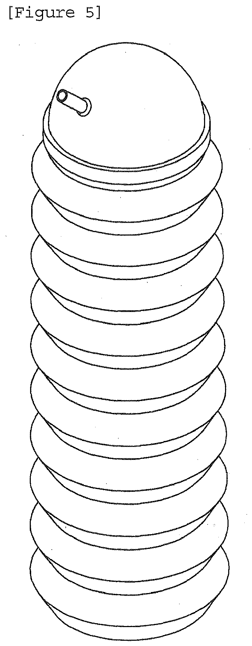

The airtight container may have a discharge port in any position. The aforementioned airtight container having the expandable and contractible means toward the direction of depth is preferred because the foam inside the main body of a container can be discharged from the discharge port by contracting the main body. The discharge port may be positioned anywhere on the airtight container; however, from the viewpoint of taking out the foam easily, it is preferably positioned on the hermetical sealing lid. An embodiment of an airtight container having an accordion structure which has a discharge port on the hermetical sealing lid is shown in Figure 5.

The material of the main body of a container and the hermetical sealing lid is not particularly limited as long as it is capable of hermetically sealing with no leaks of the liquid mixture and has enough durability against shaking for forming foam, and examples thereof include plastic and paper.

### [Anti-slip means]

The airtight container, into which each agent is charged and hermetically sealed, is shaking with a gloved hand in order to provide the liquid mixture of each agent in the state of uniform foam. Moreover, as described above, it preferably has a reasonable capacity. Accordingly, in order to prevent an airtight container from slipping out even when a small-handed user shakes the container while grabbing it with a gloved hand, the airtight container preferably has anti-slip means toward both the longitudinal and transverse directions on the surface thereof. The anti-slip means may have concave and/or convex shapes on the surface of the container, preferably convex shapes which provide easy grip to the pad of the finger when it is held. When the anti-slip means has convex shapes, from the viewpoint of giving easy grip to the pad of the finger when it is held without pain, the protrusion is preferably 0.1 to 5 mm, more preferably 0.2 to 3 mm, even more preferably 0.25 to 2.5 mm from the surface of the container. The form of the protrusion, in the longitudinal cross-sectional view in the direction of protrusion, is preferably droplet or square shape.

The anti-slip means may be provided on the entire area or a part of the side of the surface of the container. The anti-slip means may be distributed at least on the main body of a container from the viewpoint of enabling good shaking. However, considering that the lid is also opened or closed by a gloved hand, the anti-slip means may be disposed more preferably on both of the main body of a container and the hermetical sealing lid. Also, from the viewpoint of securely gripping the container, the anti-slip means is preferably provided at least at a position where the pad of the thumb is placed, more preferably at respective positions where the pads of the thumb and other fingers are placed.

Hereinafter, patterns of the anti-slip means are disclosed; however, the anti-slip means is not limited to these embodiments. Further, these patterns are not strictly distinguishable concepts from each other, while, these patterns may be used in combination.
- Concave pattern: An oval concave dent formed on the surface of the container serves as the anti-slip means by being gripped by the thumb of the dominant hand. Because the thumb plays an important role in holding the container firmly, when the thumb is securely fixed, the concave dent serves as the anti-slip means toward both the longitudinal and transverse directions. The concave dent may be of any size and depth as long as the thumb can grip it, it is preferably large and deep enough to be gripped by at least the pad of the thumb.
- Dot/oval/rectangular pattern: One dot/oval/rectangular protrusion is preferred because it serves as the anti-slip means toward both the longitudinal and transverse directions when hooked by the pad of the thumb. It is preferred that the interval, along the side of the container, of the center of these dot-like protrusions is within 2 cm because these protrusions can be hooked by the pad of the thumb more strongly. It is preferred that the interval, along the side of the container, of the center of these dot/oval/rectangular protrusions is 8 to 15 cm because these protrusions can be hooked by the pad of the thumb and the pads of other fingers. The diameter of dot-like protrusion is preferably 1 mm to 1 cm, more preferably 2 to 5 mm. The length of the largest part of an oval/rectangular protrusion is preferably 2 mm to 3 cm, more preferably 5 mm to 1 cm. The direction of the maximum length may or may not be inclined with respect to the vertical direction of the container.
- Linear pattern (1): A straight line with a total length of 1 to 12 cm which makes ±20 to ±70° angle with respect to the vertical direction of the container is also preferred since it serves as the anti-slip means toward both the longitudinal and transverse directions. It is also preferred that a plurality of lines making the same angle with respect to the vertical direction of the container are distributed over one sixteenth or more of the circumference of the side of the container at either regular or irregular intervals. It is also preferred that those lines are disposed at respective positions where the pads of the thumb and the other fingers are placed. The width of the straight line is preferably 0.1 mm to 5 mm, more preferably 0.5 mm to 3 mm.

- Curved line pattern: A wavy line with a total length of 1 to 12 cm which makes 0 to ±90° angle with respect to the vertical direction of the container is also preferred since it serves as the anti-slip means toward both the longitudinal and transverse directions. It is also preferred that a plurality of wavy lines making the same angle with respect to the vertical direction of the container are distributed over one sixteenth or more of the circumference of the side of the container at either regular or irregular intervals, and it is also similarly preferred that such lines are evenly formed all around the side. The frequency and height of the wavy line are each preferably 5 mm to 6 cm. It is also preferred that those lines are disposed at respective positions where the pads of the thumb and the other fingers are placed. The width of the straight line is preferably 0.1 mm to 5 mm, more preferably 0.5 mm to 3 mm.
- Handle pattern: A ring formed like a handle of a cup may be attached. Since the handle can be grasped, the container can be stably held, and the handle thereby serving as the anti-slip means toward both the longitudinal and transverse directions.
- Strap or band pattern: A strap or band may be attached on a part of the container, through which a finger or a hand can be inserted. When a finger or a hand is firmly fixed through the strap or band, the strap or band serves as the anti-slip means toward both the longitudinal and transverse directions. When a hand is firmly fixed between the strap or band and the container, the strap or band serves as the anti-slip means toward both the longitudinal and transverse directions.
- Linear pattern (2) (an example of a combination): It is also preferred that one or two or more of the first straight lines with a total length of 1 to 12 cm which makes 0 to ±90° angle with respect to the vertical direction of the container and one or two or more of the second straight lines which makes ±20 to 90° angle with respect to the first straight lines are distributed over one sixteenth or more of the circumference of the side of the container, and it is also similarly preferred that such lines are evenly distributed all around the side. It is also preferred that those lines are distributed at respective positions where the pads of the thumb and the other fingers are placed. It is more preferred that three or more of the first and/or second straight lines are distributed. The interval between each of the first straight lines or each of the second straight lines may be regular or irregular. It is also preferred that those lines are distributed at respective positions where the pads of the thumb and the other fingers are placed. The width of the straight line is preferably 0.1 mm to 5 mm, more preferably 0.5 mm to 3 mm.

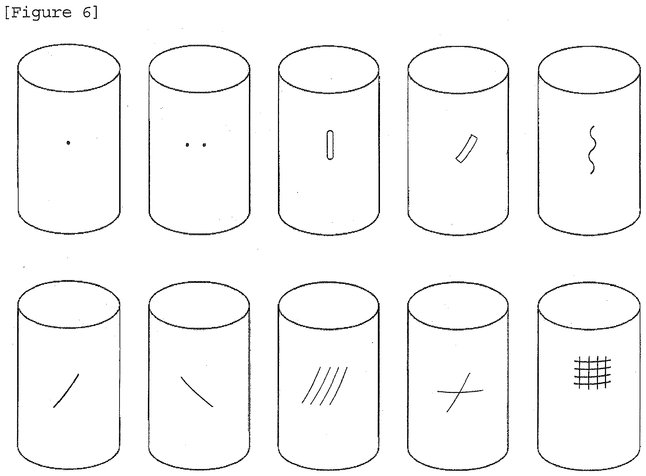

Specific examples of the above-described anti-slip means are shown in Figure 6.

### [Stirrer]

The kit for dyeing or bleaching of the present invention forms foam of a liquid mixture while mixing the first part, the second part, and the thickening composition, or mixing the alkali agent, the oxidizing agent, the thickening agent etc. contained in a molded product and the medium, by shaking. Accordingly, from the viewpoint of facilitating the mixing, it is also preferred that the kit for dyeing or bleaching of the present invention further contains a stirrer as a component of the kit.

From the viewpoint of forming an adequate amount of uniform foam while facilitating mixing, and making application of the formed foam to the hair easy, it is preferred that the stirrer moves vigorously within the foam while shaking but immediately sinks to the bottom of the main body of a container upon completion of the shaking. In view of the above, the specific gravity of the stirrer is preferably in a range of 5.0 to 8.0 g/cm³, more preferably 6.0 to 7.9 g/cm³, and even more preferably 7.0 to 7.8 g/cm³. Examples of a material having such a specific gravity include metal, preferably iron and stainless steel.

However, as the stirrer is in contact with a liquid mixture containing an alkali agent and an oxidizing agent for a certain time, the material composing the stirrer may be dissolved into the hair dye. From the viewpoint of preventing the dissolving of the material, a stirrer is more preferably a plastic-coated metal. In that case, an average specific gravity of the whole stirrer is preferably within the aforementioned range.

From the viewpoint of facilitating mixing and obtaining an adequate amount of uniform foam, the stirrer is preferably spherical, in a football shape, and so on. From the viewpoint of facilitating mixing and obtaining an adequate amount of uniform foam, the volume is preferably in the range of 0.5 to 15 cm³, more preferably 1 to 10 cm³, and even more preferably 2 to 5 cm³ per stirrer.

From the viewpoint of facilitating mixing and obtaining an adequate amount of uniform foam, the number of stirrers used in the kit for dyeing or bleaching is preferably 1 to 10, more preferably 2 to 5, and even more preferably 3 to 4.

### [Hair dyeing or bleaching method]

In order to dye or bleach the hair (particularly, head hair) using the kit for dyeing or bleaching of the present invention, it is preferable to perform the following steps (a) to (d) or steps (a) to (e).
(a) charging a first part containing an alkali agent, a second part containing an oxidizing agent (and further, a thickening composition, if present), or a molded product containing an alkali agent, an oxidizing agent, and a thickening agent in the aforementioned forms and a medium into a main body of an openable and closable airtight container,
(b) hermetically sealing the airtight container,
(c) forming foam by shaking the airtight container,
(d) taking out the formed foam and applying the foam to hair, and
(e) re-foaming the applied foam on the hair.

When applying the hair dye or bleaching agent to the hair, a glove may be worn, whenever before applying the agent. However, in consideration of the possibility that the hand gets dirty also when charging the agents in the step (a), it is preferable to wear a glove before performing the step (a). Also, it is preferable to comb hair in advance at any stage before application of the foam to the hair, in order to prevent the hair from becoming tangled during the treatment of re-foaming in the step (e), thereby inhibiting spattering of the liquid mixture. Also, routinely performed blocking is not necessary during the application of a hair dye composition, rather the blocking is preferably not performed. Omission of blocking makes the operation of applying the foam of the liquid mixture to the hair and re-foaming more easily. From the viewpoint of obtaining even hair dyeing and preventing dripping while obtaining a sufficient hair dyeing effect, preferably no hair styling product is applied to the hair to which the subject hair dye composition is to be applied upon the hair dyeing treatment. Also, from the viewpoint of preventing dilution of the liquid mixture, obtaining even hair dyeing results, while preventing dripping and obtaining a sufficient hair dyeing effect, the hair is preferably dried hair. When the hair is shampooed right before the hair dyeing treatment, it is preferable to dry the hair before performing the hair dyeing treatment.

Step (a): Before mixing, any of the first part, the second part, and a separate thickening composition may contain a thickening agent. This is a step in which each of these agents or the aforementioned molded product and medium are charged into the main body of an airtight container. Each agent may be charged in any order, or may be charged at the same time. The agents are contact with each other after these agents are charged into an airtight container; however, mixing of these agents is not essential at this stage. After the charge, these agents may be mixed by stirring in advance before hermetically sealing the container in the step (b), alternatively, these agents may be mixed at the same time as the foam is formed by shaking in the below mentioned step (c). There may be about several minutes to 10 minutes interval between completion of the step (a) and starting the subsequent step. However, considering that the reaction derived from oxidation dye proceeds and the viscosity of the system increases due to a thickening agent, it is preferable to immediately and sequentially proceed all the way to the step (d) of applying the foam to the hair upon completion of the step (a). It should be noted that the airtight container preferably has the aforementioned anti-slip means.

Step (b): This is a step in which the airtight container is hermetically sealed by mounting a hermetical sealing lid on the main body of the airtight container after charging all of the agents into the main body of the airtight container. The lid is mounted so that the content is not spilled off during shaking in the below mentioned step (c), and when the container has its own sealing mechanism, it may be sealed in accordance with the mechanism.

Step(c): This is a step in which foam is formed by shaking the airtight container. Although each of the agents which have been charged in the step (a) may be mixed in advance, from the viewpoint of simplifying the operation, the agents are preferably mixed at the same time by shaking. Although a tool may be used as means of shaking, from the viewpoint of simplifying the operation, the airtight container is preferably shaken by a hand. Although it may be shaken by one hand, from the viewpoint of securely and firmly holding the airtight container, it is preferably shaken by both hands. The position of both hands for securely holding the container can be exemplified as follows:
- Placing one hand on the hermetical sealing lid and the other hand on the bottom surface opposite to the hermetical sealing lid.
- Placing one hand on the side of the main body of a container and the other hand on the hermetical sealing lid.
- Placing both hands on the side of the main body of a container.

From the viewpoint of preventing the container from slipping out of a hand when shaking or, preventing that the lid is opened during shaking to splash the content, "placing one hand on the hermetical sealing lid and the other hand on the bottom surface opposite to the hermetical sealing lid" or "placing one hand on the side of the main body of a container and the other hand on the hermetical sealing lid" is preferred.

From the viewpoint of forming foam having fine texture and adequate foam volume, the airtight container is shaken at a rate of speed of preferably 1 to 6 rounds, more preferably 2 to 5 rounds, and even more preferably 3 to 4 rounds per second (the "round" includes one up-and-down motion). The distance of shaking is preferably 5 to 50 cm, more preferably 10 to 40 cm, and even more preferably 20 to 30 cm. From the viewpoint of efficiently forming foam having fine texture and adequate foam volume, the number of shakes is preferably 5 to 60 rounds, more preferably 10 to 50 rounds, and even more preferably 20 to 40 rounds.

Upon completion of the step (c), the hermetical sealing lid is opened and the process proceeds to the step (d).

Step (d): This is a step in which the formed foam is taken out of the airtight container and applied to the hair. The foam may be applied to the whole hair or a part of the hair. In short, where to apply the foam may be decided depending on the purpose of hair dyeing and on the amount of the liquid mixture used for hair dyeing.

When taking out the formed foam from the inside of the airtight container, the foam may be transferred to another container using some means. In that case, after being transferred to a different container, the foam will be applied to the hair by some means. Alternatively, it may be also possible to scoop the foam out of the inside of the airtight container using a tool such as a comb or brush and then apply it to the hair using the tool. Alternatively, it may also be possible to scoop the foam out of the inside of the airtight container with a hand and then directly apply the scooped foam on the hand to the hair. Using a hand is preferred because the operation of applying the foam and spreading it on the hair is easy, and because splashing the foam due to excessive amount of application per one operation can be prevented, and moreover, increasing a frequency of scooping motions caused by scooping a too little amount per one operation can be prevented.

Step (e): From the viewpoint of allowing the foam of the liquid mixture to reach deep into the root of the hair, obtaining preferable dyeability even if the foam formed by shaking has an insufficient quality, and preventing dripping during the time for which the product is left to stand, which is caused when the viscosity of the liquid mixture is reduced due to decomposition of the thickening agent or inhibition of the thickening action of the thickening agent by the components of a hair dye during the time for which the product is left to stand as described above, the foam is preferably re-foamed on the hair. The re-foaming may be performed by infusing gas or using a tool such as a vibrator and brush, or by using fingers, among these, it is preferably performed by using fingers.

The re-foaming may be performed after the foam has been completely disappeared, during disappearing of the foam, or before the applied foam make some change. Alternatively, the foam may be re-foamed after the foam has been applied to the entire area to which the foam is intended to be applied or while it is applied. In another word, the step (e) may be performed after completion of the step (d), or may be performed during the step (d). In the present invention, the viscosity of the liquid mixture may decrease or increase with time. After disappearance of the foam, dripping may occur if the viscosity is decreased, whereas if the viscosity is increased, re-foaming of foam may be difficult, this may make the spreading of the agent more difficult. Moreover, in the case if the step (c) is performed again after proceeding to the step (d), preferable foam is not easily formed because the ratio between the content volume and the capacity of the airtight container has been changed and the gloved hand is likely to be wet by the agents, which makes holding the container by hand more slippery. For the above reasons, it is preferable not to perform the step (c) once the process has proceeded to the step (d). Accordingly, from the viewpoint of solving the inconvenience caused by changes in the viscosity, it is preferable to perform the step (e) while performing the step (d), and it is more preferable to perform only the step (e) once the application of foam has been completed. Re-foaming of foam may be performed continuously once or repeated multiple times intermittently. Although the re-foaming may be performed every time that the form is taken out of the airtight container and applied to the hair, the re-foaming may be performed 2 to 10 times , more preferably 3 to 5 times, per application to the hair.

Upon completion of the above operations, the foam is left to stand on the hair. The foam is preferably left to stand for 3 to 60 minutes, more preferably 5 to 45 minutes from the initiation of application of the foam.

Upon completion of leaving the foam to stand on the hair, the liquid mixture is rinsed off. Thereafter, the hair is appropriately shampooed and conditioned, rinsed with water, and then dried.

### Examples

Hair dyes having the following formulations were prepared. It should be noted that the component whose concentration is not specifically described indicates the active amount. Formulation Example 1

| • First part (Liquid) | (% by mass) |
|---|---|
| Para-aminophenol | 0.1 |
| Toluene-2,5-diamine | 0.046 |
| 5-Amino-o-cresol | 0.23 |
| Strong aqueous ammonia (28%) | 6.0 |
| Ammonium bicarbonate | 10.5 |
| Alkyl (C10-16) glycoside | 6.16 |
| Sodium laureth sulfate | 2.7 |
| Trideceth-9 | 0.5 |
| Laureth-23 | 2.0 |
| Myristyl alcohol | 0.2 |
| Propylene glycol | 4.0 |
| Ethanol | 10.0 |
| Polyquaternium 22^{*1} | 0.4 |
| Polyquaternium 7^{*2} | 0.5 |
| Ascorbic acid | 0.4 |
| Sodium sulfite | 0.5 |
| EDTA-4Na | 0.1 |
| Perfume | 0.95 |
| Purified water | Balance |

| • Second part (liquid) | (% by mass) |
|---|---|
| Aqueous hydrogen peroxide (35%) | 16.3 |
| Sodium laureth sulfate | 0.62 |
| Cetearyl alcohol | 0.48 |
| Myristyl alcohol | 0.28 |
| Oxyquinoline sulfate | 0.04 |
| Etidronic acid | 0.08 |
| Sodium hydroxide | An amount to adjust the pH of the second part to 3.5 |
| Purified water | Balance |

| • Third part (liquid) | (% by mass) |
|---|---|
| Hydroxypropyl xanthan gum^{*3} | 2.0 |
| Ethanol | 10.0 |
| Purified water | Balance |

| | |
|---|---|
| *1: Merquat 280 (Nalco Company) *2: Merquat 550 (Nalco Company) *3: Rhaball GumEX (Dainippon Sumitomo Pharma Co., Ltd.) | |

The amount of each agent used
First part : Second part : Third part = 40 g : 60 g : 12 g The pH of the liquid mixture: 9.1

Into a hermetically sealable cylindrical plastic container having an inner diameter of 9 cm and a height of 14 cm, the first part, the second part, and the third part having the above formulations were charged all at once, and the container was shaken 30 rounds at a speed of three rounds per second with a shaking distance of 30 cm to form foam. After all of the charged liquids turned to be firm foam, the foam was left to stand for about 10 minutes; however, no separation of liquid from the foam was observed.

An individual, who does not usually use a hair dye, scoops the formed foam from the container by a gloved hand and applies it to a wig (No. 755s Beaulax Co., Ltd.) by pressing it against the wig, during which operation the individual re-foams the foam in a similar way as shampooing once every three operation of scooping out the formed foam and applying the foam to the hair. After all of the formed foam has been applied to the whole hair, the foam is left to stand for thirty minutes. Subsequently, the operation of rinsing with water, shampooing, and then conditioning was repeated twice, and then the hair was dried. The visual evaluation of the result of the hair dyeing by expert panelists confirm that even and good dyeing results are obtained.

Also, when the pads of the thumb and the index finger are securely hooked on a protrusion having a diameter of 3 mm and a height of 2 mm placed on the side of the aforementioned plastic container at a distance of one-third from top and on a protrusion having the same size placed at the opposite position with respect to the aforementioned protrusion respectively, shaking is favorably performed.

### Formulation Example 2

A hair dye having the following formulation was prepared.

| • First part (liquid) | (% by mass) |
|---|---|
| Para-aminophenol | 0.1 |
| Toluene-2,5-diamine | 0.046 |
| 5-Amino-o-cresol | 0.23 |
| Strong aqueous ammonia (28%) | 6.0 |
| Ammonium bicarbonate | 8.0 |
| Monoethanolamine | 2.4 |
| Alkyl (C10-16) glycoside | 2.0 |
| Sodium cocoyl glutamate | 6.0 |
| Sodium laureth-6 acetate | 2.0 |
| Trideceth-9 | 0.5 |
| Laureth-23 | 2.0 |
| Myristyl alcohol | 0.2 |
| Propylene glycol | 4.25 |
| Ethanol | 9.5 |
| Polyquaternium22^{*4} | 1.125 |
| EDTA-4Na | 0.1 |
| Perfume | 0.95 |
| Purified water | Balance |

| • Second part (liquid) | (% by mass) |
|---|---|
| Aqueous hydrogen peroxide (35%) | 16.3 |
| Stearyl trimonium chloride | 0.84 |
| Ceteth-40 | 0.55 |
| Cetearyl alcohol | 0.88 |
| Myristyl alcohol | 0.25 |
| Oxyquinoline sulfate | 0.04 |
| Etidronic acid | 0.08 |
| Sodium hydroxide | An amount to adjust the pH of |
| the second part to 3.5 | |
| Purified water | Balance |

| • Third part (powder) | (% by mass) |
|---|---|
| Methyl cellulose^{*5} | 100 |

| | |
|---|---|
| *4: Merquat 295 (Nalco Company) *5: Metolose SM-4000 (Shin-Etsu Chemical Co., Ltd.) | |

The amount of each agent used
First part : Second part : Third part = 40 g : 60 g : 3 g
The pH of the liquid mixture: 9.2
The viscosity of a liquid mixture of the first part and the second part: 15 mPa·s
The viscosity of a liquid mixture of the first part, the second part, and the third part: 350 mPa·s

The container and the method of forming foam employed are the same as that of Formulation Example 1. Foam having preferable usability and foaming property, which can penetrate easily and deeply into the root of the hair when pressed against the hair, is obtained.

### Formulation Example 3

A hair dye was prepared in the same formulation as Formulation Example 2, except that the third part of Formulation Example 2 was substituted with the following ingredient and was used in an amount of 17.1 g.

| • Third part (gel) | (% by mass) |
|---|---|
| Hydroxyethyl cellulose^{*6} | 6.4 |
| Purified water | Balance |

| | |
|---|---|
| *6: HEC Daicel SE850 (Daicel Corporation) | |

The viscosity of a liquid mixture of the first part and the second part: 15 mPa·s
The viscosity of a liquid mixture of the first part, the second part, and the third part: 1280 mPa·s
The pH of the liquid mixture: 9.1

Foam was formed by the same method and using the same container as above. The obtained foam was preferable to be applied by pressing it against the hair. Even after leaving the foam to stand for 30 minutes, no separation of liquid was observed.

### Formulation Example 4

A hair dye was prepared in the same formulation as Formulation Example 2, except that the third part in Formulation Example 2 was substituted with the following ingredient and was used in an amount of 19.3 g, .

| • Third part (gel) | (% by mass) |
|---|---|
| Hydroxyethyl cellulose^{*7} | 17.1 |
| Purified water | Balance |

| | |
|---|---|
| *7: HEC Daicel SE850 (Daicel Corporation) | |

The viscosity of a liquid mixture of the first part and the second part: 15 mPa·s
The viscosity of a liquid mixture of the first part, the second part, and the third part: 8600 mPa·s
The pH of the liquid mixture: 9.1

As a result of forming foam by the same method and using the same container as above, foam having preferable elasticity was obtained. Even after leaving the foam to stand for 30 minutes, no separation of liquid was observed.

### Formulation Example 5

A hair dye having the following formulation was prepared.

| • First part (powder) | (% by mass) |
|---|---|
| Toluene-2,5-diamine sulfate | 2.5 |
| Meta-aminophenol sulfate | 1.25 |
| Para-aminophenol sulfate | 1.25 |
| Resorcin | 1.25 |
| Sodium carbonate | 50.0 |
| HEDTA-3Na | 2.5 |
| β-cyclodextrin | 6.25 |
| Ammonium sulfate | 12.5 |
| Sodium lauryl sulfate | 1.25 |
| Magnesium stearate | 1.25 |
| Sodium sulfate | Balance |

| • Second part (liquid) | (% by mass) |
|---|---|
| Aqueous hydrogen peroxide (35%) | 8.2 |
| Sodium laureth sulfate | 2.7 |
| Oxyquinoline sulfate | 0.04 |
| Etidronic acid | 0.08 |
| Sodium hydroxide solution (48%) | 0.056 |
| Purified water | Balance |

| • Third part (powder) | (% by mass) |
|---|---|
| Hydroxyethyl cellulose^{*7} | 5.0 |
| Cellulose gum^{*8} | 80.0 |
| Sodium sulfate | Balance |

| | |
|---|---|
| *7: HEC Daicel SE850 (Daicel Corporation) *8: CELLOGEN P (Dai-Ichi Kogyo Seiyaku Co., Ltd.) | |

The amount of each agent used
First part : Second part : Third part = 20 g : 125 g : 5 g

By using the container used in Formulation Example 1, preferable foam is formed, and preferable results of hair dyeing are obtained.

### Formulation Example 6

In accordance with the procedure described in Example 1 of JP-A-2003-518028, molded products of hair dye, 25 tablets, each weighing 4 g, are produced. Into the airtight container used in Formulation Example 1, 100 g of purified water and 25 tablets of molded products of hair dye, produced as above, are charged at once, and the container is shaken 30 rounds at a speed of three rounds per second with a shaking distance of 30 cm to form foam. All the charged compositions are dissolved and the resulting liquid turns to be an elastic, firm foam, and even after the formed foam is left to stand for about 10 minutes, no separation of liquid is observed.

The formed foam is scooped out of the container by a gloved hand and applied to a wig (No. 755s Beaulax Co., Ltd.) by pressing it against the wig. The foam is re-foamed in a similar way as shampooing once every three times of the operation of scooping out the foam and applying the resulting foam to the hair. After all of the formed foam is applied to the entire hair, the foam is left to stand for 30 minutes. Subsequently, the operation of rinsing with water, shampooing, and then conditioning is repeated twice, and then the hair is dried. Even when the above operation is performed by an individual who does not usually use a hair dye, the visual evaluation of the result of the hair dyeing by expert panelists confirm that even and preferable dyeing results are obtained.

## Claims

1. A kit for hair dyeing or bleaching comprising:
a first part comprising an alkali agent,
a second part comprising an oxidizing agent,
a thickening composition comprising a thickening agent, and
an openable and closable airtight container,
wherein the first and second parts and the thickening composition are charged into the container and a mixture thereof is shaken to form foam of the mixture,
wherein any one or more of the first part, the second part, and the thickening composition comprise a surfactant, and
wherein the inner circumference of the opening of the main body of the container is 25 to 35 cm.

2. The kit for hair dyeing or bleaching according to claim 1, wherein the airtight container is equipped with anti-slip means for prevention of slipping in both longitudinal and transverse directions on the surface thereof.

3. The kit for hair dyeing or bleaching according to claim 1 or 2, wherein the thickening composition is in the form of liquid, gel, or jelly.

4. A kit for hair dyeing or bleaching comprising:
a first part comprising an alkali agent,
a second part comprising an oxidizing agent, and
an openable and closable airtight container, into which the first and second parts are charged so as to form foam of a mixture of the first and second parts by shaking to form foam of the mixture, the airtight container being equipped with anti-slip means for prevention of slipping in both longitudinal and transverse directions on the surface thereof,
wherein any one or more of the first part and the second part comprise a surfactant,
and wherein the inner circumference of the opening of the main body of the container is 25 to 35 cm.

5. The kit for hair dyeing or bleaching according to claim 4, wherein at least one of the first and second parts comprises a thickening agent and is in the form of liquid, gel, or jelly.

6. The kit for hair dyeing or bleaching according to any one of claims 1 to 5, wherein a total concentration of the surfactant in the liquid mixture is 0.01 to 20% by mass.

7. The kit for hair dyeing or bleaching according to any one of claims 1 to 6, wherein a viscosity of the liquid mixture (25°C) is 50 to 20,000 mPa·s.

8. The kit for hair dyeing or bleaching according to any one of claims 1 to 7, wherein pH of the liquid mixture (25°C) is 7 to 12.

9. The kit for hair dyeing or bleaching according to any one of claims 1 to 8, wherein the general shape of a main body of the airtight container is cylindrical shape.

10. The kit for hair dyeing or bleaching according to any of claims 1 to 9, wherein the volume of the airtight container is 100 to 2000 mL.

11. The kit for hair dyeing or bleaching according to any of claims 1 to 10, wherein the airtight container comprises an expandable and contractible means in the depth direction.

12. The kit for hair dyeing or bleaching according to claim 11, wherein the expandable and contractible means is an accordion structure.

13. The kit for hair dyeing or bleaching according to claim 11 or 12, wherein the airtight container comprises a discharge port which is positioned at any position.

14. The kit for hair dyeing or bleaching according to claim 13, wherein the discharge port is positioned on a lid of the airtight container.

15. The kit for hair dyeing or bleaching according to any of claims 1 to 14, further comprising a stirrer.

16. A method for dyeing or bleaching hair, comprising the following steps (a) to (d):
(a) charging all of a first part comprising an alkali agent, a second part comprising an oxidizing agent, and a thickening composition comprising a thickening agent into a main body of an openable and closable airtight container,
(b) hermetically sealing the airtight container,
(c) forming foam by shaking the airtight container, and
(d) taking out the formed foam from the airtight container and applying the foam to hair,
wherein any one or more of the first part, the second part, and the thickening composition comprise a surfactant, and wherein the inner circumference of the opening of the main body of the container is 25 to 35 cm.

17. A method for dyeing or bleaching hair, comprising the following steps (a) to (d):
(a) charging both of a first part comprising an alkali agent and a second part comprising an oxidizing agent into a main body of an openable and closable airtight container,
(b) hermetically sealing the airtight container,
(c) forming foam of a liquid mixture by shaking the airtight container, and
(d) taking out the formed foam from the airtight container and applying the foam to hair,
wherein the airtight container is equipped with anti-slip means for prevention of slipping in both longitudinal and transverse directions on the surface thereof,
wherein any one or more of the first part and the second part comprise a surfactant,
and wherein the inner circumference of the opening of the main body of the container is 25 to 35 cm.

## Patentansprüche

1. Set zum Färben oder Bleichen von Haaren, umfassend:
einen ersten Bestandteil, umfassend ein Alkalimittel,
einen zweiten Bestandteil, umfassend ein Oxidationsmittel,
eine Verdickungszusammensetzung, umfassend ein Verdickungsmittel, und
einen luftdichten Behälter, der geöffnet und geschlossen werden kann,
worin der erste und der zweite Bestandteil und die Verdickungszusammensetzung in den Behälter gefüllt werden und die Mischung davon geschüttelt wird, so dass ein Schaum aus der Mischung gebildet wird,
worin irgendeines oder mehr des ersten Bestandteils, des zweiten Bestandteils und der Verdickungszusammensetzung ein Tensid umfassen, und
worin der Innenumfang der Öffnung des Grundkörpers des Behälters 25 bis 35 cm ist.

2. Set zum Färben oder Bleichen von Haaren gemäß Anspruch 1, worin der luftdichte Behälter mit Antirutschmitteln versehen ist, so dass ein Abrutschen auf dessen Oberfläche sowohl in Längs- als auch in Querrichtung verhindert wird.

3. Set zum Färben oder Bleichen von Haaren gemäß Anspruch 1 oder 2, worin die Verdickungszusammensetzung in der Form einer Flüssigkeit, eines Gels oder eines Gelees ist.

4. Set zum Färben oder Bleichen von Haaren, umfassend:
einen ersten Bestandteil, umfassend ein Alkalimittel,
einen zweiten Bestandteil, umfassend ein Oxidationsmittel,
eine Verdickungszusammensetzung, umfassend ein Verdickungsmittel, und
einen luftdichten Behälter, der geöffnet und geschlossen werden kann und in den der erste und der zweite Bestandteil gefüllt werden, so dass ein Schaum aus der Mischung des ersten und des zweiten Bestandteils gebildet wird, worin der luftdichte Behälter mit Antirutschmitteln versehen ist, so dass ein Abrutschen auf dessen Oberfläche sowohl in Längs- als auch in Querrichtung verhindert wird,
worin irgendeines oder mehr des ersten Bestandteils, des zweiten Bestandteils und der Verdickungszusammensetzung ein Tensid umfassen, und
worin der Innenumfang der Öffnung des Grundkörpers des Behälters 25 bis 35 cm ist.

5. Set zum Färben oder Bleichen von Haaren gemäß Anspruch 4, worin mindestens einer des ersten und des zweiten Bestandteils ein Verdickungsmittel umfasst und in der Form einer Flüssigkeit, eines Gels oder eines Gelees ist.

6. Set zum Färben oder Bleichen von Haaren gemäß mindestens einem der Ansprüche 1-5, worin die Gesamtkonzentration des Tensids in der flüssigen Mischung 0,01 bis 20 Massen-% ist.

7. Set zum Färben oder Bleichen von Haaren gemäß mindestens einem der Ansprüche 1-6, worin die Viskosität der flüssigen Mischung 50 bis 20.000 mPa·s (25°C) ist.

8. Set zum Färben oder Bleichen von Haaren gemäß mindestens einem der Ansprüche 1-7, worin der pH (25°C) der flüssigen Mischung 7 bis 12 ist.

9. Set zum Färben oder Bleichen von Haaren gemäß mindestens einem der Ansprüche 1-8, worin die Grundform des Grundkörpers eine zylindrische Form ist.

10. Set zum Färben oder Bleichen von Haaren gemäß mindestens einem der Ansprüche 1-9, worin das Volumen des luftdichten Behälters 100 bis 2000 mL ist.

11. Set zum Färben oder Bleichen von Haaren gemäß mindestens einem der Ansprüche 1-10, worin der luftdichte Behälter ein ausdehnbares und zusammenziehbares Hilfsmittel in der Tiefenrichtung umfasst.

12. Set zum Färben oder Bleichen von Haaren gemäß Anspruch 11, worin das ausdehnbare und zusammenziehbare Hilfsmittel eine Ziehharmonika-Struktur aufweist.

13. Set zum Färben oder Bleichen von Haaren gemäß Anspruch 11 oder 12, worin der luftdichte Behälter einen Druckstutzen umfasst, der an irgendeiner Stelle positioniert ist.

14. Set zum Färben oder Bleichen von Haaren gemäß Anspruch 13, worin der Druckstutzen auf einem Deckel des luftdichten Behälters angeordnet ist.

15. Set zum Färben oder Bleichen von Haaren gemäß mindestens einem der Ansprüche 1-14, ferner umfassend einen Rührer.

16. Verfahren zum Färben oder Bleichen von Haaren, umfassend die folgenden Schritte (a) bis (d):
(a) Füllen eines ersten Bestandteils, umfassend ein Alkalimittel, eines zweiten Bestandteils, umfassend ein Oxidationsmittel, und einer Verdickungszusammensetzung, umfassend ein Verdickungsmittel, in einen Grundkörper eines luftdichten Behälters, der geöffnet und geschlossen werden kann,
(b) hermetisches Verschließen des luftdichten Behälters,
(c) Bilden eines Schaums durch Schütteln des luftdichten Behälters, und
(d) Entnehmen des gebildeten Schaums aus dem luftdichten Behälter und Auftragen des Schaums auf das Haar,
worin irgendeines oder mehr des ersten Bestandteils, des zweiten Bestandteils und der Verdickungszusammensetzung ein Tensid umfassen, und
worin der Innenumfang der Öffnung des Grundkörpers des Behälters 25 bis 35 cm ist.

17. Verfahren zum Färben oder Bleichen von Haaren, umfassend die folgenden Schritte (a) bis (d):
(a) Füllen eines ersten Bestandteils, umfassend ein Alkalimittel, und eines zweiten Bestandteils, umfassend ein Oxidationsmittel, in einen Grundkörper eines luftdichten Behälters, der geöffnet und geschlossen werden kann,
(b) hermetisches Verschließen des luftdichten Behälters,
(c) Bilden eines Schaums aus einer flüssigen Mischung durch Schütteln des luftdichten Behälters,
(d) Entnehmen des gebildeten Schaums aus dem luftdichten Behälter und Auftragen des Schaums auf das Haar,
worin der luftdichte Behälter mit Antirutschmitteln versehen ist, so dass ein Abrutschen auf dessen Oberfläche sowohl in Längs- als auch in Querrichtung verhindert wird,
worin irgendeines oder mehr des ersten Bestandteils und des zweiten Bestandteils ein Tensid umfassen, und
worin der Innenumfang der Öffnung des Grundkörpers des Behälters 25 bis 35 cm ist.

## Revendications

1. Kit de teinture ou décoloration capillaire comprenant :
une première partie comprenant un agent alcalin,
une seconde partie comprenant un agent oxydant,
une composition épaississante comprenant un agent épaississant, et
un récipient étanche à l'air pouvant être ouvert et fermé,
dans lequel les première et seconde parties et la composition épaississante sont chargées dans le récipient et un mélange de celles-ci est agité pour former une mousse du mélange,
dans lequel une quelconque ou plusieurs des première partie, seconde partie et composition épaississante comprend un tensioactif, et
dans lequel la circonférence interne de l'ouverture du corps principal du récipient est de 25 à 35 cm.

2. Kit de teinture ou de décoloration capillaire selon la revendication 1, dans lequel le récipient étanche à l'air est équipé de moyens anti-glissement pour empêcher un glissement à la fois dans les directions longitudinale et transversale sur la surface de celui-ci.

3. Kit de teinture ou de décoloration capillaire selon la revendication 1 ou 2, dans lequel la composition épaississante se présente sous la forme de liquide, de gel ou de gelée.

4. Kit de teinture ou de décoloration capillaire comprenant :
une première partie comprenant un agent alcalin,
une seconde partie comprenant un agent oxydant, et
un récipient étanche à l'air pouvant être ouvert et fermé, dans lequel les première et seconde parties sont chargées de manière à former une mousse d'un mélange des première et seconde parties en agitant pour former une mousse du mélange, le récipient étanche à l'air étant équipé de moyens anti-glissement pour empêcher un glissement à la fois dans les directions longitudinale et transversale sur la surface de celui-ci,
dans lequel une quelconque ou plusieurs des première partie et seconde parties comprend un tensioactif,
et dans lequel la circonférence interne de l'ouverture du corps principal du récipient est de 25 à 35 cm.

5. Kit de teinture ou de décoloration capillaire selon la revendication 4, dans lequel au moins une des première et seconde parties comprend un agent épaississant et se présente sous la forme de liquide, de gel ou de gelée.

6. Kit de teinture ou de décoloration capillaire selon l'une quelconque des revendications 1 à 5, dans lequel une concentration totale du tensioactif dans le mélange liquide est de 0,01 à 20 % en masse.

7. Kit de teinture ou de décoloration capillaire selon l'une quelconque des revendications 1 à 6, dans lequel une viscosité du mélange liquide (25 °C) est de 50 à 20 000 mPa·s.

8. Kit de teinture ou de décoloration capillaire selon l'une quelconque des revendications 1 à 7, dans lequel le pH du mélange liquide (25 °C) est de 7 à 12.

9. Kit de teinture ou de décoloration capillaire selon l'une quelconque des revendications 1 à 8, dans lequel la forme générale d'un corps principal du récipient étanche à l'air est une forme cylindrique.

10. Kit de teinture ou de décoloration capillaire selon l'une quelconque des revendications 1 à 9, dans lequel le volume du récipient étanche à l'air est de 100 à 2000 mL.

11. Kit de teinture ou de décoloration capillaire selon l'une quelconque des revendications 1 à 10, dans lequel le récipient étanche à l'air comprend des moyens extensibles et contractables dans la direction de profondeur.

12. Kit de teinture ou de décoloration capillaire selon la revendication 11, dans lequel les moyens extensibles et contractables sont constitués d'une structure en accordéon.

13. Kit de teinture ou de décoloration capillaire selon la revendication 11 ou 12, dans lequel le récipient étanche à l'air comprend un orifice de décharge qui est positionné à n'importe quelle position.

14. Kit de teinture ou de décoloration capillaire selon la revendication 13, dans lequel l'orifice de décharge est positionné sur un couvercle du récipient étanche à l'air.

15. Kit de teinture ou de décoloration capillaire selon l'une quelconque des revendications 1 à 14, comprenant en outre un agitateur.

16. Procédé de teinture ou de décoloration capillaire, comprenant les étapes (a) à (d) suivantes :
(a) charger la totalité d'une première partie comprenant un agent alcalin, d'une seconde partie comprenant un agent oxydant, et d'une composition épaississante comprenant un agent épaississant dans un corps principal d'un récipient étanche à l'air pouvant être ouvert et fermé,
(b) sceller hermétiquement le récipient étanche à l'air,
(c) former de la mousse en secouant le récipient étanche à l'air, et
(d) retirer la mousse formée du récipient étanche à l'air et appliquer la mousse sur les cheveux,
dans lequel une quelconque ou plusieurs de la première partie, la seconde partie, et la composition épaississante comprend un tensioactif,
et dans lequel la circonférence interne de l'ouverture du corps principal du récipient est de 25 à 35 cm.

17. Procédé de teinture ou de décoloration capillaire, comprenant les étapes (a) à (d) suivantes :
(a) charger à la fois une première partie comprenant un agent alcalin et une seconde partie comprenant un agent oxydant dans un corps principal d'un récipient étanche à l'air pouvant être ouvert et fermé,
(b) sceller hermétiquement le récipient étanche à l'air,
(c) former une mousse à partir d'un mélange liquide en secouant le récipient étanche à l'air, et
(d) retirer la mousse formée du récipient étanche à l'air et appliquer la mousse sur les cheveux,
dans lequel le récipient étanche à l'air est équipé de moyens anti-glissement pour empêcher un glissement à la fois dans les directions longitudinale et transversale sur la surface de celui-ci,
dans lequel une ou plusieurs de la première partie et la seconde partie comprend un tensioactif,
et dans lequel la circonférence intérieure de l'ouverture du corps principal du récipient est de 25 à 35 cm.
